# EUROPÄISCHE PATENTSCHRIFT

(11) **EP 0 602 401 B1**
(45) Veröffentlichungstag und Bekanntmachung des Hinweises auf die Patenterteilung: **03.09.1997**
(21) Anmeldenummer: 93118525.0
(22) Anmeldetag: 17.11.1993
(51) Int. Cl.: A61G 12/00, H01R 25/14

(54) **Trägervorrichtung für medizinische Apparate**
Support device for medical apparatus
Dispositif de support pour appareils médicaux

(30) Priorität: 26.11.1992 DE 4239625
(43) Veröffentlichungstag der Anmeldung: 22.06.1994
(73) Patentinhaber: Fresenius AG, 61350 Bad Homburg v.d.H (DE)
(72) Erfinder: Neuder, Klaus, Dr., D-63179 Obertshausen (DE)
(74) Vertreter: Fuchs, Luderschmidt & Partner Patentanwälte

(56) Entgegenhaltungen:
- EP-A- 0 453 725
- EP-A- 0 499 660
- US-A- 3 781 567

## Beschreibung

Die Erfindung betrifft eine Trägervorrichtung für medizinische Apparate nach dem Oberbegriff des Anspruchs 1.

Eine derartige Trägervorrichtung ist aus der DE-C- 40 30 368 bekannt. Mit dieser gattungsgemäßen Trägervorrichtung werden medizinische Apparate, z. B. in Form von Infusionsgeräten kabellos aufgestellt. Das größte Anwendungsgebiet liegt derzeit in der Patientenüberwachung und im infusionstherapeutischen Bereich. Grundsätzlich ist die Trägervorrichtung mit einer Kupplungseinrichtung versehen und der medizinische Apparat ist mit einer Klemmeinrichtung ausgestattet.

Diese bekannt Trägervorrichtung weist zur Halterung mehrerer Infusionsgeräte eine Säule auf, auf die die Infusionsgeräte, die u-förmig ausgebildet sind, aufgeschoben werden. An der Säule sind leistenförmige Träger- und Kupplungselemente angebracht. Die aufschiebbaren Infusionsgeräte weisen nutartige Führungs- und Steckerelemente auf. Die mechanische Verbindung von Säule und Infusionsgeräten wird über die leistenförmigen Trägerelemente und entsprechende nutartige Führungselemente in den Infusionsgeräten hergestellt. Die elektrische Verbindung erfolgt über die Kupplungs- und entsprechende Steckerelemente. Die elektrische Stromversorgung der Infusionsgeräte erfolgt vom Inneren des Geräteträgers aus. Zusätzlich können die Infusionsgeräte, falls nicht Die Erfindung betrifft eine Trägervorrichtung für medizinische Apparate nach dem Oberbegriff des Anspruchs 1.

Eine derartige Trägervorrichtung ist aus der DE-C-40 30 368 bekannt. Mit dieser gattungsgemäßen Trägervorrichtung werden medizinische Apparate, z. B. in Form von Infusionsgeräten kabellos aufgestellt. Das größte Anwendungsgebiet liegt derzeit in der Patientenüberwachung und im infusionstherapeutischen Bereich. Grundsätzlich ist die Trägervorrichtung mit einer Kupplungseinrichtung versehen und der medizinische Apparat ist mit einer Klemmeinrichtung ausgestattet.

Diese bekannte Trägervorrichtung weist zur Halterung mehrerer Infusionsgeräte eine Säule auf, auf die die Infusionsgeräte, die u-förmig ausgebildet sind, aufgeschoben werden. An der Säule sind leistenförmige Träger- und Kupplungselemente angebracht. Die aufschiebbaren Infusionsgeräte weisen nutartige Führungs- und Steckerelemente auf. Die mechanische Verbindung von Säule und Infusionsgeräten wird über die leistenförmigen Trägerelemente und entsprechende nutartige Führungselemente in den Infusionsgeräten hergestellt. Die elektrische Verbindung erfolgt über die Kupplungs- und entsprechende Steckerelemente. Die elektrische Stromversorgung der Infusionsgeräte erfolgt vom Inneren des Geräteträgers aus. Zusätzlich können die Infusionsgeräte, falls nicht am Geräteträger aufgeschoben, mit einem wiederaufladbaren Akkumulator versehen werden. Dieser Akkumulator wird bei Bedarf auf das Infusionsgerät aufgeschoben.

Eine weitere Trägervorrichtung ist aus der DE-34 02 885 bekannt und weist eine freistehende Versorgungssäule auf, die als Geräteträger ausgebildet ist. An dieser Säule sind vertikale, paarweise an der Vorder- und Rückseite der Säule verlaufende Profilschienen angebracht, in denen Konsolen, Halter und Zubehörteile verschiebbar und mittels Gleitverriegelungen arretierbar sind. Durch diese Kopplungselemente erfolgt die mechanische Verbindung von Säule und medizinischen Apparaten. Weiterhin enthält die Säule Fluid-Leitungen und elektrische Leitungen, so daß auch hier die Stromversorgung der medizinischen Apparate vom Inneren des Gerätes aus erfolgt, nachdem die elektrische Leitung des medizinischen Apparates an den entsprechenden Versorgungsauslaß der Säule angeschlossen wurde.

Diese gattungsgemäßen Trägervorrichtungen sind jedoch insofern nachteilig, als die mechanische und elektrische Verbindung mit hoher Genauigkeit hergestellt werden muß. Die elektrischen und mechanischen Verbindungselemente können erst nach einem gegenseitigen Ausrichten miteinander verbunden werden. Mit anderen Worten ist das Herstellen einer Verbindung zwischen Geräteträger und Infusionsgerät zeitaufwendig. Die schnelle Einsatzfähigkeit der gattungsgemäßen Trägervorrichtungen wird deshalb durch das komplizierte Kupplungsverfahren sehr beeinträchtigt, was in Notfällen hinderlich ist.

Es ist daher Aufgabe der vorliegenden Erfindung, eine Trägervorrichtung der im Oberbegriff des Anspruchs 1 angegebenen Art zu schaffen, welche größere Freizügigkeit bei der Anordnung der medizinischen Geräte bei einfachem und ausfallsicherem Aufbau ermöglicht.

Die Lösung dieser Aufgabe erfolgt durch die Merkmale des Anspruchs 1.

Die Kupplungseinrichtung der erfindungsgemäßen Trägervorrichtung weist zwei elektrisch voneinander isolierte Leiterschienen auf, die durchgehend entlang der Längserstreckung des Trägers verlaufen. Diese Leiterschienen gewähren die Stromversorgung bzw. Datenübertragung und die mechanische Befestigung von zumindest einem medizinischen Apparat. Das heißt, daß, betreffend den elektrischen Teil, die Stromversorgung, die Datenübertragung und der Personalaufruf komplett über die Leiterschienen erfolgt. Hierdurch ergibt sich der Vorteil, daß in großem Maße auf mehrfache und getrennte Kabelleitungen verzichtet werden kann, wodurch der benötigte Vorbereitungsaufwand für den Einsatz der medizinischen Apparate weitgehend verringert wird.

Ebenfalls erlauben die sich durchgehend entlang der Längsrichtung erstreckenden Leiterschienen eine stufenlose Befestigung und Verschiebung von mehreren medizinischen Apparaten. Außerdem besteht ein weiterer Vorteil darin, daß die medizinischen Geräte an jeder beliebigen Stelle an den Schienen befestigt und verschoben werden können, ohne dafür die Apparate von den Leiterschienen entfernen zu müssen.

Die EP-A- 0 453 725 beschreibt eine Stromschiene mit von außen abgreifbaren Stromleitern und einem Trägerkörper aus Glas für die Stromleiter. Für das mechanische Tragen von äußeren Apparaten im Sinne der Gattung der Erfindung ist dieser Glasträgerkörper nicht vorgesehen und auch nicht geeignet. Ebenso werden keine Daten über die Stromleiter übertragen. Es liegt lediglich eine spezielle Stromsammelschiene vor, die mit mechanisch separat gehalterten und geführten elektrischen Verbrauchern nur elektrisch zwecks Stromzuführung mittels Schleifkontakten in Verbindung steht.

Die EP-A-0 499 660 beschreibt einen Energie und Informationen übertragenden Bus, an den über einen Busankoppler elektronische Endgeräte anschließbar sind. Die Schrift wendet sich allein an einen modularen Aufbau des Busankopplers im Rahmen eines Systems zur Übertragung von Informationen über Netzanlagen an mehrere Endverbraucher in einer Großanlage, das mit einer Trägervorrichtung für medizinische Apparate nicht vergleichbar ist.

Die Unteransprüche haben vorteilhafte Weiterbildungen der Erfindung zum Inhalt.

Bei einer bevorzugten Ausführungsform sind die Leiterschienen der Trägervorrichtung als glatte Metallschienen ausgebildet. Hierdurch ergibt sich der Vorteil, daß die Leiterschienen leichter reinigbar sind und die Trägervorrichtung den hygienischen Erfordernissen entspricht.

Bei einer weiteren bevorzugten Ausführungsform sind die Leiterschienen der Trägervorrichtung über Koppelelemente miteinander verbunden, so daß eine weitere Verbindung von Leiterschienen untereinander ermöglicht wird.

Bei einer besonders bevorzugten Ausführungsform weist die Trägervorrichtung eine Spannungs- und Datenaustauscheinrichtung auf, die an der Basis des Trägers angeordnet ist. Hieraus resultiert der Vorteil, daß eine zentrale Steuerung und Überwachung von mehreren an der Trägervorrichtung angeschlossenen medizinischen Apparaten möglich wird.

Bei einer besonders bevorzugten Ausführungsform ist die Spannungs- und Datenaustauscheinrichtung als Stromversorgungs- und Businterface ausgebildet. Diese Einrichtung erlaubt die gleichzeitige Stromversorgung und den Datentransfer zwischen den angeschlossenen medizinischen Geräten und einem Computer.

Um eine Steuerung, bzw. Eingabe von Daten und eine interaktive grafische Datenverarbeitung zu ermöglichen, kann die Spannungs- und Datenaustauscheinrichtung mit einem Computer oder PC verbunden werden. Ebenfalls ist die Spannungs-Datenaustauscheinrichtung mit einer Stromquelle oder einem Stromversorgungsnetz verbindbar, damit die Stromversorgung sichergestellt ist.

Die Erfindung betrifft ferner einen medizinischen Apparat in Kombination mit einer Trägervorrichtung nach dem Anspruch 1. Der Apparat weist vorzugsweise eine Klemmvorrichtung mit zwei elektrischen Kontakten aufweist, die mit den Leiterschienen kontaktierbar sind, so daß die elektrische Verbindung ohne zusätzliche Kabelverbindung erfolgt.

Vorteilhafterweise weist die Klemmvorrichtung zwei gegenüber angeordnete Klemmbacken auf, die mittels einer Klemmschraube oder einer anderen geeigneten Klemmeinrichtung betätigbar sind. Hierdurch ergibt sich der weitere Vorteil, daß bei der mechanischen Arretierung gleichzeitig der elektrische Kontakt erfolgt, daß heißt in einem einfachen Arbeitsvorgang wird eine mechanische und elektrische Verbindung hergestellt. Ebenfalls ermöglicht eine solche Klemmeinrichtung ein leichtes Verschieben des medizinischen Apparates entlang der Leiterschienen, ohne daß die elektrische Verbindung unterbrochen wird.

Bei einer besonders bevorzugten Ausführungsform weist der medizinische Apparat einen integrierten wiederaufladbaren Akkumulator auf, was den Vorteil ergibt, daß der medizinische Apparat ständig über eine individuelle aufgeladene Stromquelle verfügt. Bei einer Netzausfallüberbrückung, bzw. einem mobilen Einsatz des medizinischen Apparates während eines Patiententransportes ist somit die Stromversorgung gewährleistet. Darüberhinaus kann aus Umweltgründen von Primärzellen zur Stromversorgung der Infusionsapparate vorteilhafterweise abgesehen werden.

Bei einer weiteren bevorzugten Ausführungsform ist der medizinische Apparat mit einem in der Klemmeinrichtung integrierten Umsetzer ausgestattet, was den Vorteil ergibt, daß unterschiedliche Datenübertragungstechniken angewendet werden können, z.B. im Fall eines Notbetriebs.

Vorteilhafterweise ist die Klemmvorrichtung ein separates Bauteil, das über eine Kabelverbindung mit dem medizinischen Apparat verbunden werden kann, um ein einfaches Austauschen der Klemmvorrichtung z.B. nach einer Störung zu ermöglichen. Auch könnte die Klemmeinrichtung als integrales Bauteil des medizinischen Apparates ausgebildet sein.

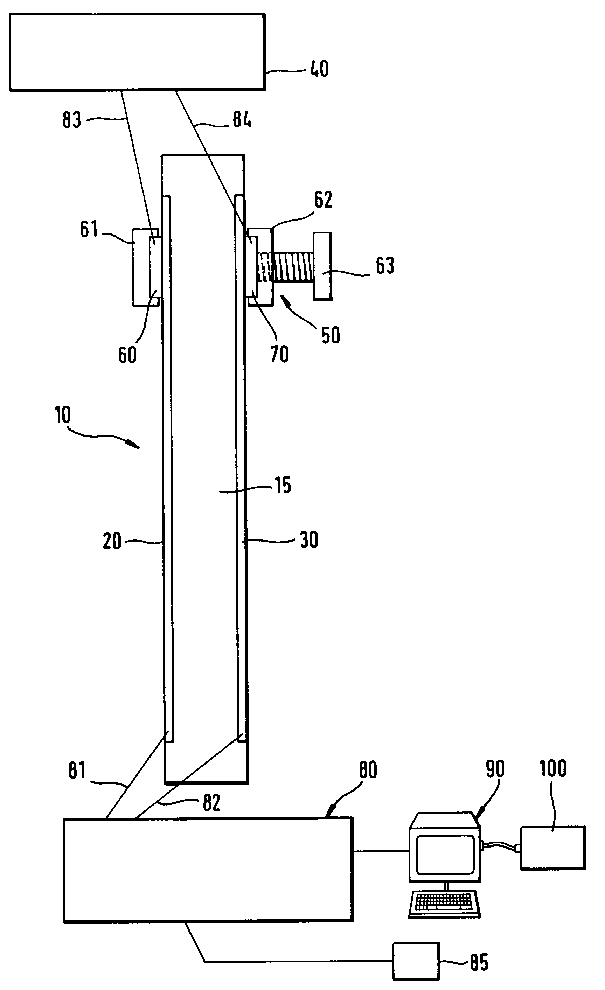

Weitere Einzelheiten, Merkmale und Vorteile der Erfindung ergeben sich aus nachfolgender Beschreibung eines Ausführungsbeispiels anhand der Zeichnung.

Die einzige Figur der Zeichnung zeigt eine schematische Darstellung einer erfindungsgemäßen Trägervorrichtung 10. Die Trägervorrichtung 10 weist einen langgestreckten (vorzugsweise säulenartigen) Träger 15 und zumindestens zwei Leiter- bzw. Stromschienen 20 und 30 auf. Vorteilhafterweise sind die Leiterschienen 20, 30 glatt und leicht reinigbar ausgebildet. Die Leiterschienen, über die die Strom- und Datenversorgung läuft, sind elektrisch voneinander isoliert angebracht. Die Leiterschienen 20, 30 können über nicht dargestellte Koppelelemente miteinander verbunden werden.

Ferner ist an der Trägervorrichtung 10 zumindestens ein medizinischer Apparat 40 befestigt. Es können aber serienmäßig mehrere medizinische Apparate an der Trägervorrichtung 10 angeschlossen werden.

Der medizinische Apparat 40 ist beispielsweise mit einer separaten Klemmeinrichtung bzw. Befestigungsklemme oder einem Halter 50 versehen, der über Leitungen 83 und 84 mit dem medizinischen Apparat verbunden ist. Mittels zwei einander gegenüber angeordneter Klemmbacken 61 und 62 ist die Klemmeinrichtung stufenlos an der Trägervorrichtung 10 befestigbar. Der medizinische Apparat 40 ist dadurch leicht entlang der Leiterschienen 20, 30 der Trägervorrichtung 10 zu verschieben.

Zur Herstellung der mechanischen und elektrischen Verbindung an den Leiterschienen 20, 30 verfügt die Klemmeinrichtung über elektrische Kontakte 60 und 70. Mittels einer Klemmschraube 63 werden die Klemmbacken und die entsprechenden Kontakte an die Leiterschienen 20, 30 der Trägervorrichtung 10 geklemmt. Ebenfalls kann die Befestigungsklemme einen nicht dargestellten Umsetzer für ein Busprotokoll mit der internen Bezeichnung RS-232-CAMUS des Patentinhabers aufweisen, damit der serienmäßige Anschluß das voneinander unabhängige Funktionieren der einzelnen angeschlossenen medizinischen Apparate ermöglicht.

Kurze nicht dargestellte Verbindungskabel stellen die elektrische Verbindung zwischen dem medizinischen Apparat 40 und dem Umsetzer her. Die Stromversorgung und die Elektronik für die Datenaufbereitung, ein intelligenter Datenkonzentrator, befinden sich in einer Spannungs- und Datenaustauscheinrichtung 80, z. B. im unteren Bereich des Trägers 15. Diese Einrichtung 80 ist über Leitungen 81, 82 mit den Leiterschienen 20, 30 verbunden. Von ihr aus führen ferner Verbindungen zum Computer 90 und zur Netzversorgung 85. Der Datenkonzentrator der Einrichtung 80 stellt dabei ein Interface zwischen den Datentleitungen des Computers und dem Datenbussystem her, damit die einzelnen medizinischen Apparate selektiv angesprochen werden können. Damit der Funktionsverlauf des medizinischen Apparates 40 beobachtet werden kann, ist ein Bedienungsmodul 100 mit dem Computer 90 verbunden.

## Patentansprüche

1. Trägervorrichtung (10) für medizinische Apparate (40)
- mit einem langgestreckten Träger (15) zum mechanischen Anklemmen und Haltern der medizinischen Apparate;
- mit einer Kupplungseinrichtung zum lösbaren Verbinden der medizinischen Apparate mit einer Stromversorgung (85) und einer Datenverarbeitung (90),
dadurch gekennzeichnet, daß
- die Kupplungseinrichtung trägerseitig zwei elektrisch voneinander isolierte Leiterschienen (20, 30) aufweist, die durchgehend entlang der Längserstreckung des Trägers (15) an seiner Außenseite verlaufen, an die die medizinischen Apparate (40) mittels einer angepaßten Klemmeinrichtung (50) mechanisch und elektrisch anklemmbar sind.

2. Trägervorrichtung nach Anspruch 1, dadurch gekennzeichnet, daß die Leiterschienen (20, 30) als Metallschienen ausgebildet sind.

3. Trägervorrichtung nach Anspruch 1 oder 2, dadurch gekennzeichnet, daß die Leiterschienen (20, 30) glatt sind.

4. Trägervorrichtung nach einem der Ansprüche 1 bis 3, dadurch gekennzeichnet, daß die Leiterschienen (20, 30) über Koppelelemente miteinander verbunden sind.

5. Trägervorrichtung nach einem der Ansprüche 1 bis 4, gekennzeichnet durch eine Spannungs- und Datenaustauscheinrichtung (80), die am Träger (15) angeordnet ist.

6. Trägervorrichtung nachAnspruch 5, dadurch gekennzeichnet, daß die Spannungs- und Datenaustauscheinrichtung (80) als Stromversorgungs- und Businterface ausgebildet ist.

7. Trägervorrichtung nach Anspruch 5 oder 6, dadurch gekennzeichnet, daß die Spannungs- und Datenaustauscheinrichtung (80) mit einem Computer (90) und einer Stromquelle (95) verbindbar ist.

8. Medizinischer Apparat (40) in Kombination mit einer Trägervorrichtung nach dem Anspruch 1, gekennzeichnet durch eine mechanisch stufenlos verstellbare und elektrisch leitende Klemmeinrichtung (50).

9. Medizinischer Apparat nach Anspruch 8, dadurch gekennzeichnet, daß die Klemmeinrichtung (50) zwei elektrische Kontakte (60, 70) aufweist, die mit den Leiterschienen (20, 30) in Kontakt bringbar sind.

10. Medizinischer Apparat nach Anspruch 8 oder 9, dadurch gekennzeichnet, daß die Klemmeinrichtung (50) zwei gegenüber angeordnete Klemmbacken (61, 62) aufweist, die mittels einer Klemmschraube (63) betätigbar sind.

11. Medizinischer Apparat nach einem der Ansprüche 8 bis 10, gekennzeichnet durch einen integrierten Akkumulator zur Stromversorgung.

12. Medizinischer Apparat nach einem der Ansprüche 8 bis 11, gekennzeichnet durch einen integrierten Umsetzer, der in der Klemmvorrichtung (50) angeordnet ist.

13. Medizinischer Apparat nach einem der Ansprüche 8 bis 12, dadurch gekennzeichnet, daß die Klemmeinrichtung (50) ein separates Bauteil ist, das über eine Kabelverbindung (83, 84) mit dem medizinischen Apparat (40) verbindbar ist.

14. Medizinischer Apparat nach einem der Ansprüche 8 bis 12, dadurch gekennzeichnet, daß die Klemmeinrichtung (50) ein integrales Bauteil des medizinischen Apparates darstellt.

## Claims

1. Stand (10) for medical apparatus (40)
- with a longitudinal support for mechanical clamping and bracketing of medical apparatus;
- with a coupling device for the removable connection of medical apparatus to a power supply (85) and a data processing system (90),
characterised by the fact that
- the coupling unit has two conductor rails (20,30), electrically isolated from each other, on the stand side, which run the full length of the stand (15) on the outside, to which the medical apparatus (40) can be mechanically and electrically clamped by means of a suitable clamping device (50).

2. Stand as per Claim 1, characterised by the fact that the conductor rails (20, 30) are designed as metal rails.

3. Stand as per Claim 1 or 2, characterised by the fact that the conductor rails (20, 30) are smooth.

4. Stand as per one of the Claims 1 to 3, characterised by the fact that the conductor rails (20, 30) are connected to one another via coupling elements.

5. Stand as per one of the Claims 1 to 4, characterised by a current and data transfer unit (80) which is accommodated on the stand (15).

6. Stand as per Claim 5, characterised by the fact that the current and data transfer unit (80) is designed as a power supply and bus interface.

7. Stand as per Claim 5 or 6, characterised by the fact that the current and data transfer unit (80) may connected to a computer (90) and a power source (95).

8. Medical apparatus (40) in combination with a stand as per Claim 1, characterised by a clamp arrangement (50) which mechanically is infinitely adjustable and which is power-conducting.

9. Medical apparatus as per Claim 8, characterised by the fact that the clamp unit (50) has two electrical contacts (60, 70) which can be placed in contact with the conductor rails (20, 30).

10. Medical apparatus as per Claim 8 or 9, characterised by the fact that the clamp device (50) has two clamping jaws (61, 62) opposite one another, which can be activated by means of a clamping screw (63).

11. Medical apparatus as per one of Claims 8 to 10, characterised by a built-in accumulator for the power supply.

12. Medical apparatus as per one of Claims 8 to 11, characterised by a built-in converter, which is accommodated in the clamp device (50).

13. Medical apparatus as per one of Claims 8 to 12, characterised by the fact that the clamp device (50) is a separate component, which can be connected via a cable connection (83, 84) to the medical apparatus (40).

14. Medical apparatus as per one of Claims 8 to 12, characterised by the fact that the clamp arrangement (50) is an integral part of the medical apparatus.

## Revendications

1. Dispositif de support (10) pour appareils médicaux (40), comportant
- un support allongé (15) pour serrer et retenir mécaniquement des appareils médicaux;
- un dispositif d'accouplement pour relier de façon détachable les appareils médicaux à une source d'alimentation en courant (85) et à une unité de traitement de données (90),
caractérisé en ce que
- le dispositif d'accouplement comporte, du côté du support, deux rails conducteurs (20,30) électriquement isolés l'un de l'autre, qui s'étendent continûment sur l'étendue en longueur du support (15) sur sa face extérieure, sur laquelle les appareils médicaux (40) peuvent être serrés mécaniquement et raccordés électriquement à l'aide d'un dispositif de serrage adapté (50).

2. Dispositif de support selon la revendication 1, caractérisé en ce que les rails conducteurs (20,30) sont agencés sous la forme de rails métalliques.

3. Dispositif de support selon la revendication 1 ou 2, caractérisé en ce que les rails conducteurs (20,30) sont lisses.

4. Dispositif de support selon l'une des revendications 1 à 3, caractérisé en ce que les rails conducteurs (20,30) sont reliés entre eux par l'intermédiaire d'éléments de couplage.

5. Dispositif de support selon l'une des revendications 1 à 4, caractérisé par un dispositif (80) d'application de tension et d'échange de données, qui est disposé sur le support (15).

6. Dispositif de support selon la revendication 5, caractérisé en ce que le dispositif (80) d'application de tension et d'échange de données est agencé sous la forme d'une interface d'alimentation en courant et de bus.

7. Dispositif de support selon la revendication 5 ou 6, caractérisé en ce que le dispositif (80) d'application de tension et d'échange de données peut être relié à un ordinateur (90) et à une source de courant (95).

8. Appareil médical (40) en combinaison avec un dispositif de support selon la revendication 1, caractérisé par un dispositif de serrage (50) réglable mécaniquement de façon progressive et électriquement conducteur.

9. Appareil médical selon la revendication 8, caractérisé en ce que le dispositif de serrage (50) comporte deux contacts électriques (60,70), qui peuvent être placés en contact avec les rails conducteurs (20,30).

10. Appareil médical selon la revendication 8 ou 9, caractérisé en ce que le dispositif de serrage (5) comporte deux mâchoires de serrage (61,62), qui sont disposées en vis-à-vis et peuvent être actionnées à l'aide d'une vis de serrage (63).

11. Appareil médical selon l'une des revendications 8 à 10, caractérisé par un accumulateur intégré pour l'alimentation en courant.

12. Appareil médical selon l'une des revendications 8 à 11, caractérisé par un convertisseur intégré, qui est disposé dans le dispositif de serrage (50).

13. Appareil médical selon l'une des revendications 8 à 12, caractérisé en ce que le dispositif de serrage (50) est un composant séparé, qui peut être relié au moyen d'une liaison à câbles (83,84) à l'appareil médical (40).

14. Appareil médical selon l'une des revendications 8 à 12, caractérisé en ce que le dispositif de serrage (50) est un composant faisant partie intégrante de l'appareil médical.
